# EUROPEAN PATENT APPLICATION

(11) **EP 1 361 221 A1**
(43) Date of publication of application: **12.11.2003**
(21) Application number: 03252706.1
(22) Date of filing: 29.04.2003
(51) Int. Cl.: C07D 277/48

(54) **A process for the preparation of a combination of famotidine polymorphs A and B**

(30) Priority: 02.05.2002 IN MU03942002; 02.05.2002 IN MU03952002
(71) Applicant: M/s. TONIRA PHARMA LIMITED, Vadodara - 390 003 Guiarat (IN)
(72) Inventor: Sriraman, Mandayam Chakravarthy, c/o M/s Tonira, Alkapuri, Vadodara - 390 003- Gujarat (IN); Vyas, Jignesh Harikesh, c/o M/s Tonira, Alkapuri, Vadodara - 390 003- Gujarat (IN); Talia, Yogen Hasmukhbhai, c/o M/s Tonira, Alkapuri, Vadodara - 390 003- Gujarat (IN); Sanyal, Janardhan Prasad, c/o M/s Tonira, Alkapuri, Vadodara - 390 003- Gujarat (IN); Shah, Mahesh Natwarlal, c/o M/s Tonira, Alkapuri, Vadodara - 390 003- Gujarat (IN)
(74) Representative: Muir, Benjamin M. J. (GB)

(57) **Abstract**

A process for the preparation of a combination of Famotidine [Chemical Name (N-Sulfamyl -3-(2-guanidinothiazole-4-yl-methylthio) propionamidine] Polymorphs A and B is disclosed.

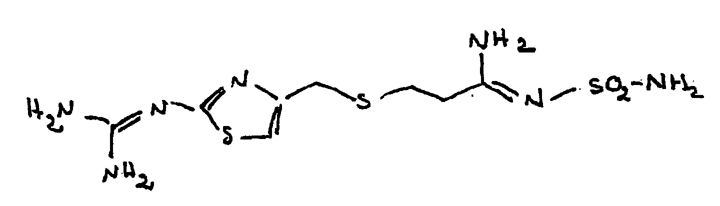

## Description

The present invention relates to a process for the preparation of a combination of Famotidine Polymorphs A and B. The full chemical name for Famotidine is N-Sulfamyl - 3-(2-guanidinothiazole-4-yl-methylthio) proionamidine].

Famotidine is a competitive inhibitor of histamine H2-receptors. The primary clinically important pharmacological activity of Famotidine is inhibition of gastric secretion. Famotidine suppresses both the acid concentration and volume of gastric secretion, while changes in pepsin secretion are proportional to volume output.

There are two polymorphs of Famotidine reported as Polymorph "A" and Polymorph "B" and their manufacturing process has been reported by M/s. Richter Gedeon, Hungary in the US Patents 4,894,459 (Jan.16, 1990), 5,120,850 (Jun.9 1992), 5,128,477 (Jul.7 1992), Canadian Patent 1265809 (Feb 13, 1990), European Patent EP 256747 ( Nov, 11, 1992) and Japanese Patent 7316141 (Feb 24 , 1988). The Famotidine Polymorph "A" and Famotidine Polymorph "B" differ in their I.R Spectra, Differential Scanning Calorimetry (DSC) Measurement Data, X-Ray Diffraction Data and Solubility data.

The process for the preparation of the individual Famotidine Polymorph "A" and Polymorph "B", use a crystallization method from water or water-alcohol mixtures, with a varying rate of cooling as described in the above referred prior art Patents.

Polymorph A of Famotidine is more stable and has low dissolution properties, while Famotidine Polymorph B is metastable and has higher dissolution properties.

One object of this invention is to provide a process for the preparation of a combination of Famotidine Polymorphs A and B whereby the dissolving rates can be controlled and the desired solubility properties of the Famotidine can be achieved.

Another object of this invention is to provide a process for the preparation of a combination of Famotidine Polymorphs A and B wherein Polymorph A and Polymorph B are of a specific ratio as desired.

According to a first aspect of the present invention, a process for the preparation of a combination of Famotidine [Chemical Name (N-Sulfamyl - 3-(2-guanidinothiazole-4-yl-methylthio) proionamidine] Polymorphs A and B is provided comprising the following steps:-
(a) Dissolving Famotidine crude in solvent, preferably methanol, under heating and stirring to form a solution:
(b) Filtering the solution of step (a), preferably to obtain a clear colourless solution;
(c) Cooling the solution of step (b) with ice and salt mixture under agitation;
(d) Seeding the cooled solution of step (c) with a mixture of Famotidine Polymorph A and Fomotidine Polymorph B for crystalisation;
(e) Filtering out the crystals of Famotidine Polymorphs A and B;
(f) Drying the crystals of Famotidine Polymorphs A and B , preferably in an oven.

The crude Famotidine is preferably prepared by an organic synthetic process.

The Famotidine (crude) to solvent ratio is preferably 1:50 to 1:70.

The Famotidine solution of step (a) is preferably treated with activated carbon.

The Famotidine solution of step (a) is preferably heated to a boiling point of 60 to 75 degrees centigrade.

At step (b) the Famotidine solution is preferably filtered in a Buchner funnel.

At step (c) the solution is preferably cooled to a temperature of 15 to 25 degrees centigrade.

The seeding mixture of Famotidine Polymorph A and Famotidine Polymorph B is preferably in a ratio of 20:80 to 50:50.

At step (f) the crystals of Famotidine Polymorphs A and B are preferably dried at a temperature of 50 to 60 degrees centigrade.

The ratio of Polymorph A to Polymorph B in the combination of Famotidine Polymorphs produced preferably varies from 15:85 to 35:65.

In an embodiment of the process for the preparation of a combination of Famotidine Polymorphs A and B according to the present invention the starting material is Famotidine (crude), which is manufactured by a synthetic process, having the total impurities and hence not suited for the use as a Pharmaceutical product. The crude Famotidine is dissolved in pure Methanol in the ration of 50 to 70 times by volume to Famotidine (crude) and crystallized by cooling in ice-sale mixture, followed by seeding at specific temperature to get the desired Polymorph A and B ratio. The seeding used is prepared by a specific process, wherein a Polymorphs mixture is obtained.

According to a second aspect of the present invention, a process based on evaporative crystallisation for the preparation of a combination of Famotidine [Chemical Name (N-Sulfamyl - 3-(2-guanidinothiazole-4-yl-methylthio) proionamidine] Polymorphs A and B is provided comprising the following steps:-
(a) dissolving Famotidine(crude) in solvent, preferably one such as methanol, under heating and stirring to form a solution, preferably followed by adding Activated Carbon to the solution and then maintaining the solution of at 45°C to 58°C for 30 minutes;
(b) filtering the solution of step (a), preferably to give a clear and colourless solution;
(c) distilling out the solvent by heating under vacuum to get a crystalline slurry, which is a combination of Famotidine Polymorph "A" and Famotidine Polymorph "B";
(d) filtering the crystalline slurry of step(c) to give the wet crystalline product, which is dried at to give a combination of Famotidine Polymorphs "A" and Famotidine Polymorph "B".

The crude Famotidine is preferably prepared by an organic synthetic process.

The Famotidine (crude) to solvent ratio is preferablyl :50 to 1:70.

At Step (b) the Famotidine solution is preferably filtered in a Buchner funnel.

At Step (c) the distilling out of solvent is preferably effected at a temperature of 45°C to 58°C.

At Step (c) the quantity of distilled solvent preferably ranges from 33% to 90% by volume.

At Step (d) the crystalline slurry of the combination of Famotidine Polymorph "A" and Famotidine Polymorph "B" is preferably filtered in a Buchner Funnel.

The crystals of Famotidine Polymorphs A and B in step (d) are preferably dried in an oven at a temperature of 50 to 60 degrees centigrade(°C).

The ratio of Polymorph A to Polymorph B in the combination of Famotidine Polymorphs produced preferably varies from 05:95 to 40:60.

In an embodiment of the process for the preparation of a combination of Famotidine Polymorphs A and B according to the present invention the starting material is Famotidine (crude), which is manufactured by a synthetic process, having different impurities and hence not suited for the use as a Pharmaceutical product. The crude Famotidine is dissolved in pure Methanol in the ratio of 50 to 70 times by volume to Famotidine (crude) and concentrated by evaporative crystallization (Distillation of solvent such as methanol by heating under vacuum) at a temperature ranging from 45°C to 58°C under vacuum to give a combination of Famotidine Polymorph "A" and Famotidine Polymorph "B".

According to a third aspect of the present invention, a process based on evaporative crystallisation for the preparation of a combination of Famotidine [Chemical Name (N-Sulfamyl - 3-(2-guanidinothiazole-4-yl-methylthio) proionamidine] Polymorphs A and B is provided comprising the following steps:
(a) Dissolving Famotidine crude in solvent, preferably one such as methanol, under heating and stirring with of Hydrochloric acid to give Famotidine Hydrochloride, which is filtered;
(b) Dissolving the Famotidine hydrochloride in solvent, preferably one such as methanol, by heating to give a Solution, preferably followed by adding Activated Carbon to the solution and maintaining the solution at 45°C to 58°C for 30 minutes;
(c) Filtering the solution, preferably to give a clear colourless solution;
(d) Making the Famotidine hydrochloride solution basic with triethylamine;
(e) Distilling out the solvent by heating under vacuum to give a crystalline slurry comprising a combination of Famotidine Polymorph"A" and Famotidine Polymorph "B";
(f) Filtering out the wet crystals of Famotidine Polymorphs A and B ;
(g) Drying the crystals of Famotidine Polymorphs A and B, preferably in an oven.

The crude Famotidine is preferably prepared by an organic synthetic process.

At Step (a) the crystalline Famotidine Hydrochloride is preferably filtered in a Buchner funnel.

At Step (b) Famotidine hydrochloride to solvent ratio is preferably 1:50 to 1:70.

At Step (c) the solution is preferably filtered in Buchner funnel.

At Step (e) the distilling out of solvent is preferably effected at a temperature of 45°C to 58°C.

At Step (e) the quantity of distilled solvent preferably ranges from 33% to 90% by volume.

At Step (f) the crystalline slurry of the combination of Famotidine Polymorph "A" and Famotidine Polymorph "B" is preferably filtered in a Buchner Funnel.

At Step (g) the crystals of Famotidine Polymorphs A and B are preferably dried at a temperature of 50 to 60 degrees centigrade.

The ratio of Polymorph A to Polymorph B in the combination of Famotidine Polymorphs produced preferably varies from 05:95 to 40:60.

In an embodiment of the process for the preparation of a combination of Famotidine Polymorphs A and B according to the present invention the starting material is Famotidine (crude), which is manufactured by a synthetic process, having different impurities and hence not suited for the use as a Pharmaceutical product. The crude Famotidine is treated with Hydrochloric acid in pure Methanol to give Famotidine Hydrochloride. Famotidine Hydrochloride is dissolved solvent such as methanol in the ratio of 50 to 70 times by volume to Famotidine hydrochloride to give a clear solution, which was Activated carbon treated to give a solution of Famotidine hydrochloride in methanol. The above said solution is made basic by adding Triethylamine and concentrated by evaporative crystallization at a temperature ranging from 45°C to 58°C under vacuum to give a combination of FamotidinePolymorph A and Famotidine Polymorph B.

According to a fourth aspect of the present invention, a process for the preparation of a combination of Famotidine Polymorphs A and B is provided comprising the following steps:
a) dissolving Famotidine crude in a solvent under heating and stirring to form a solution;
b) filtering the solution of step (a);
c) cooling the solution of step (b) with an ice and salt mixture under agitation;
d) seeding the cooled solution of step (c) with a mixture of Famotidine polymorph A and Famotidine Polymorph B for crystallization;
e) filtering out the crystals of Famotidine Polymorphs A and B;
f) drying the crystals of Famotidine Polymorphs A and B.

The crude Famotidine is preferably prepared by an organic synthetic process.

The Famotidine (crude) to solvent ratio is preferably 1:50 to 1:70.

The Famotidine solution of step (a) is preferably treated with activated carbon.

The Famotidine solution of step (a) is preferably heated to a boiling point of 60 to 75 degrees centigrade.

At step (b) the Famotidine solution is preferably filtered in a Buchner funnel.

At step (c) the solution is preferably cooled to a temperature of 15 to 25 degrees centigrade.

The seeding mixture of Famotidine Polymorph A and Famotidine Polymorph B is preferably in a ratio of 20:80 to 50:50.

At step (f) the crystals of Famotidine Polymorphs A and B are preferably dried at a temperature of 50 to 60 degrees centigrade.

The ratio of Polymorph A to Polymorph B in the combination of Famotidine Polymorphs produced preferably varies from 15:85 to 35:65.

According to a fifth aspect of the present invention a process for the preparation of a combination of Famotidine Polymorphs A and B is provided comprising the following steps:
(a) dissolving Famotidine crude in a solvent under heating and stirring to form a solution;
(b) filtering the solution of step (a);
(c) concentrating the solution by heating under vacuum to get a crystalline slurry;
(d) filtering out the crystals of Famotidine Polymorphs A and B;
(e) drying the crystals of Famotidine Polymorphs A and B.

The crude Famotidine is preferably prepared by an organic synthetic process.

The Famotidine (crude) to solvent ratio is preferably 1:50 to 1:70.

Activated carbon is preferably added to the solution at step (a) before the solution is filtered in step (b).

At step (b) the Famotidine solution is preferably filtered in a Buchner funnel.

At step (c) the solution is preferably concentrated at a temperature around 45°C to 58°C.

At step (e) the crystals of Famotidine Polymorphs A and B are preferably dried at a temperature of 50 to 60 degrees centigrade.

The ratio of Polymorph A to Polymorph B in the combination of Famotidine Polymorphs produced preferably varies from 05:95 to 40:60.

According to a sixth aspect of the present invention, a process for the preparation of a combination of Famotidine Polymorphs A and B is provided comprising the following steps:
(a) dissolving Famotidine crude in a solvent under heating and stirring and reacting with concentrated Hydrochloric acid to give Famotidine Hydrochloride;
(b) dissolving the Famotidine Hydrochloride in solvent;
(c) filtering the solution of step (b);
(d) making the solution of step (c) basic with triethylamine;
(e) concentrating the solution of step (d) by heating under vacuum to give a crystalline slurry;
(f) filtering out the crystals of Famotidine Polymorphs A and B;
(g) drying the said crystals of Famotidine Polymorphs A and B.

The crude Famotidine is preferably prepared by an organic synthetic process.

The Famotidine Hydrochloride to solvent ratio is preferably 1:50 to 1:70.

Activated carbon is preferably added to the solution of step (b) before the solution is filtered in step (c).

At step (c) the Famotidine Hydrochloride solution is preferably filtered in a Buchner funnel.

At step (e) the solution is preferably concentrated at a temperature of around 45°C to 58°C.

At step (g) the crystals of Famotidine Polymorphs A and B are preferably dried at a temperature of 50 to 60 degrees centigrade.

The ratio of Polymorph A to Polymorph B in the combination of Famotidine Polymorphs produced preferably varies from 05:95 to 40:60.

Table 1 illustrates the nature of polymorph obtained by different crystallization process. In order to determine the ratio of Polymorph "A" and Polymorph "B", the Differential Scanning Calorimetry technique (DSC analysis) was used to produce the data generated from the following experiments. Other techniques like solubility determination, I R Spectra and X-ray diffraction are not suitable for determining the ratio of the individual polymorphs.

**Table 1**

| **No.** | **Famotidine (Crude)** | **Solvent** | **Ratio (Wt:Volume)** | **Dissolution Temperature** | **Crystallisation Method** | **Polymorph Ratio** |
|---|---|---|---|---|---|---|
| 1. | 50 g | Methanol | 1:55 | 65 C | Cooling water | Polymorph "A" |
| 2. | 50 g | Methanol | 1:55 | 65 C | Ice + Salt mixture | Polymorph "B" |
| 3. | 50 g | Methanol | 1:55 | 65 C | Ice + Salt mixture Seeding at 20 C Ratio 20:80 | Polymorph "A" 22% Polymorph "B" 78% |
| 4. | 50 g | Methanol | 1:60 | 65 C | Ice + Salt mixture Seeding at 25 C Ratio (20:80) | Polymorph "A" 35% Polymorph "B" 65% |
| 5. | 50 g | Methanol | 1:60 | 65 C | Ice + Salt mixture Seeding at 15 C Ratio (50:50) | Polymorph "A" 15% Polymorph "B" 85% |
| 6. | 50 g | Methanol | 1:55 | | Evaporative crystallization 33 % solvent distilled out | Polymorph" A" 05% Polymorph "B" 95% |
| 7. | 50 g | Methanol | 1:60 | | Evaporative Crystallisation method 90% solvent distilled out | Polymorph "A" 27% Polymorph "B" 73% |
| 8. | 50 g (Famotidine Hydrochloride) | Methanol | 1:60 | | Evaporative crystallization 70% solvent distilled out | Polymorph "A" 42% Polymorph "B" 58% |

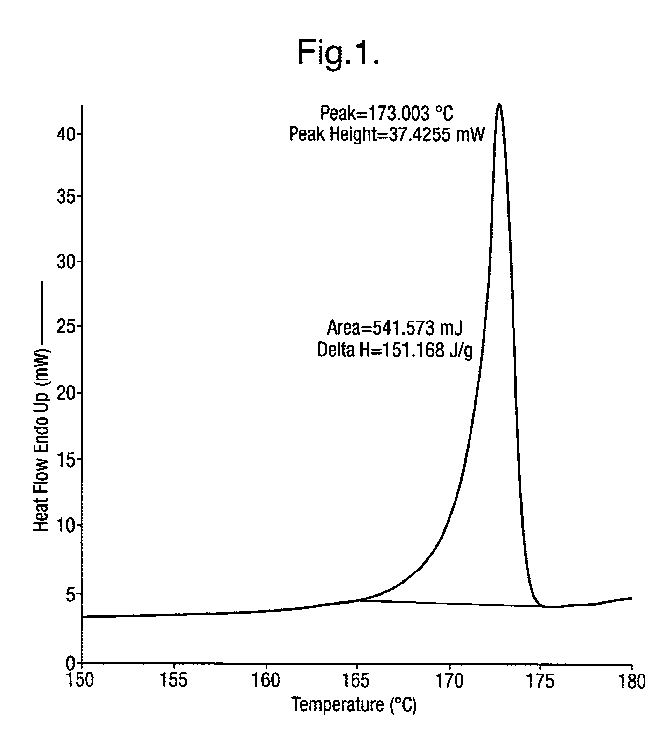

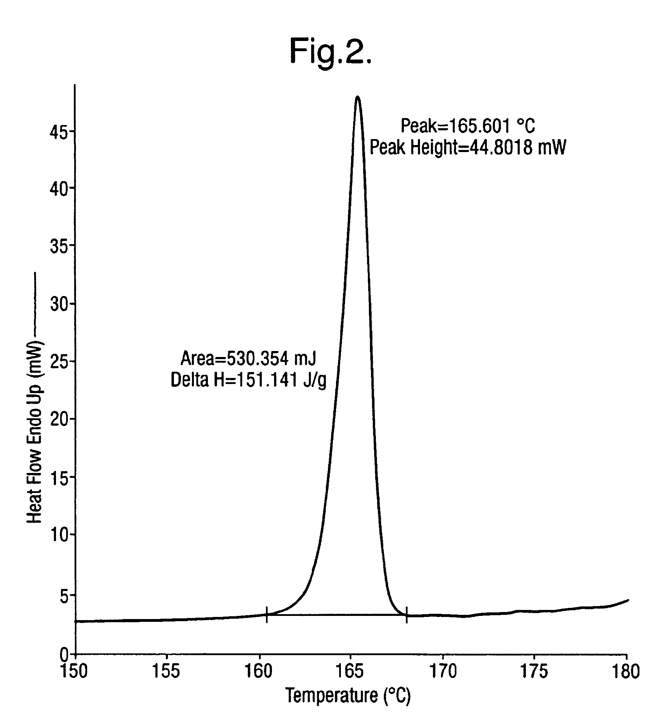

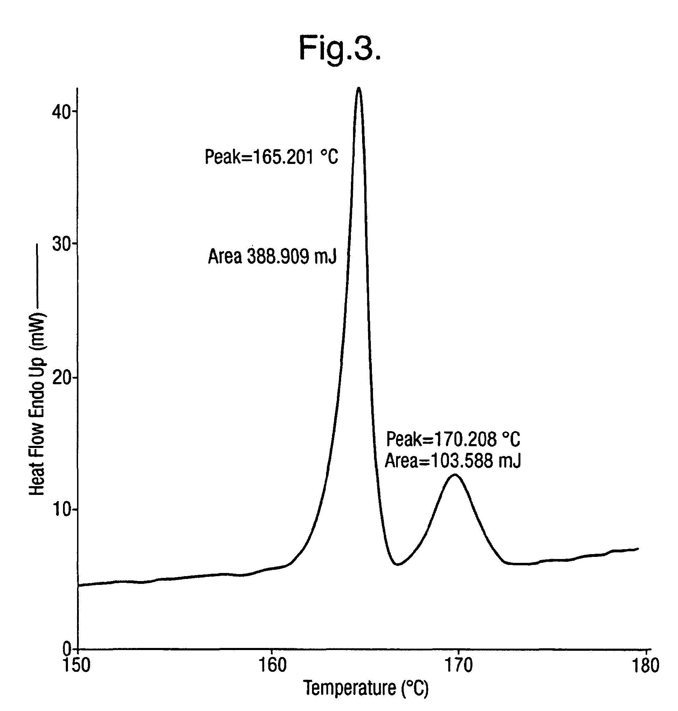

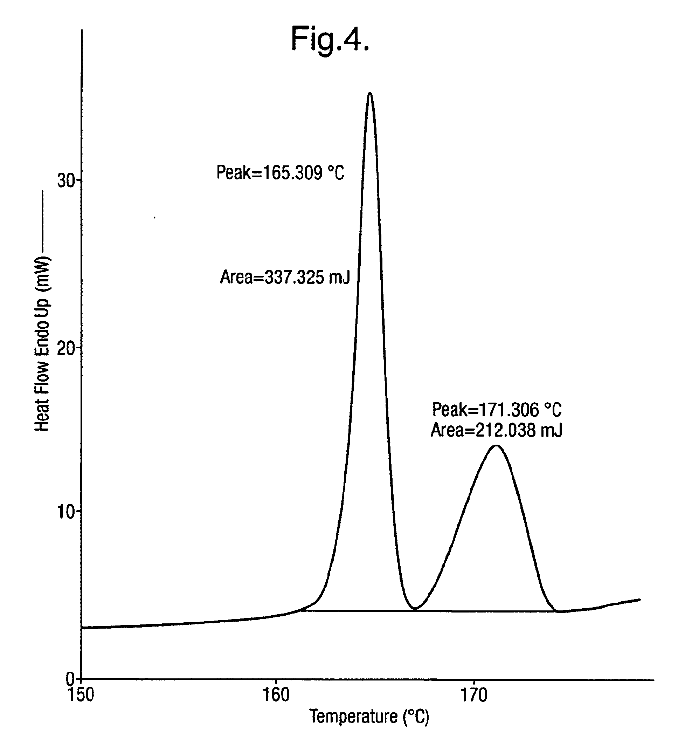

In the accompanying drawings Fig.1 illustrates the DSC analysis indicating Famotidine pure polymorph A.
In the accompanying drawings Fig.2 illustrates the DSC analysis indicating Famotidine pure polymorph B.
In the accompanying drawings Fig.3 illustrates the DSC analysis indicating a combination of Famotidine polymorph A and polymorph B in a specific ratio of 22:78.
In the accompanying drawings Fig.4 illustrates the DSC analysis indicating a combination of Famotidine polymorph A and polymorph B in a specific ratio of 35:65.

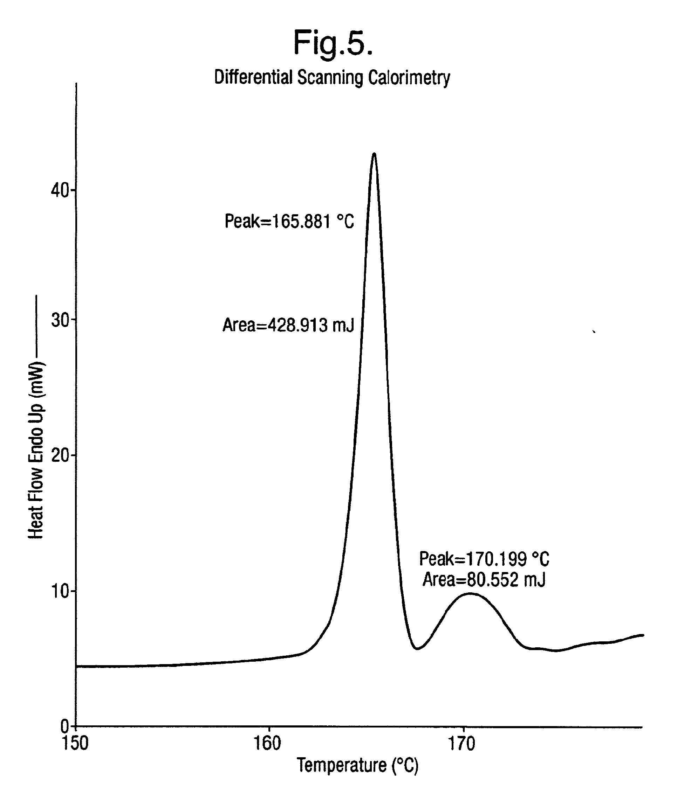

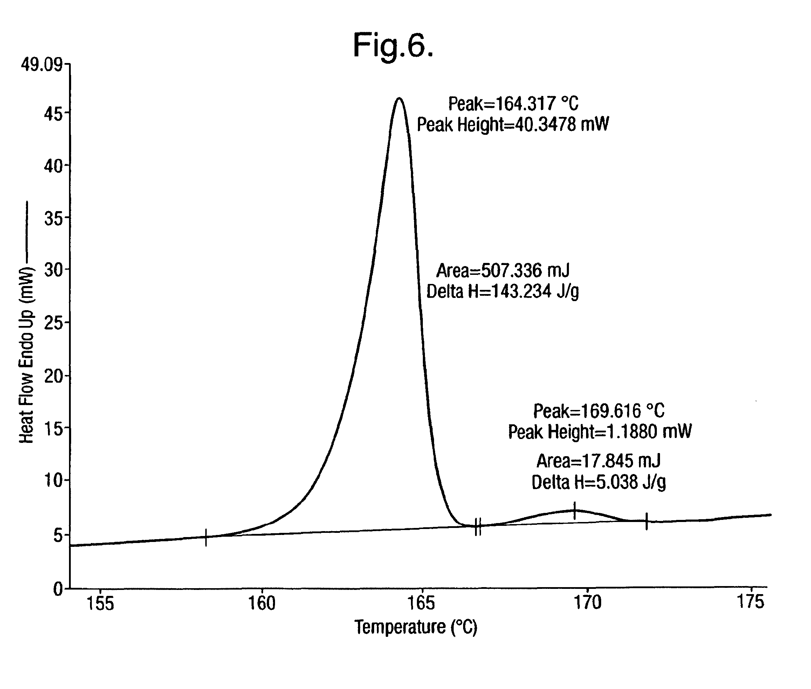

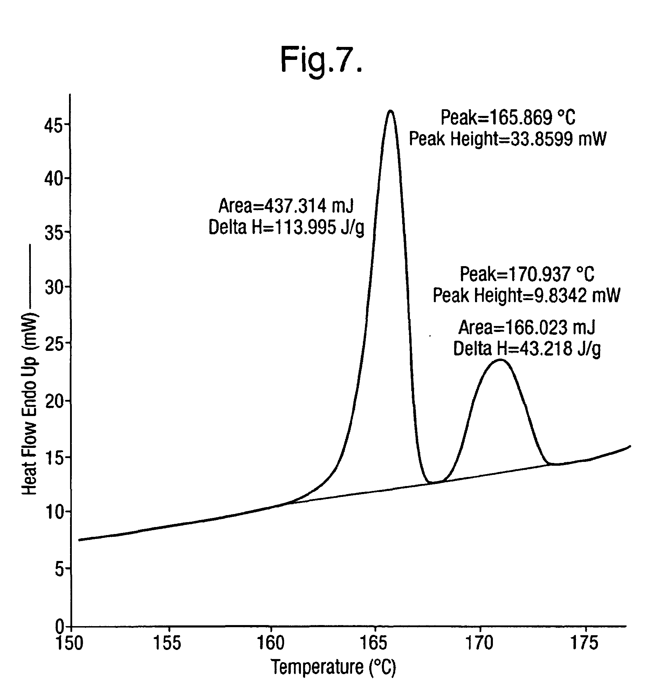

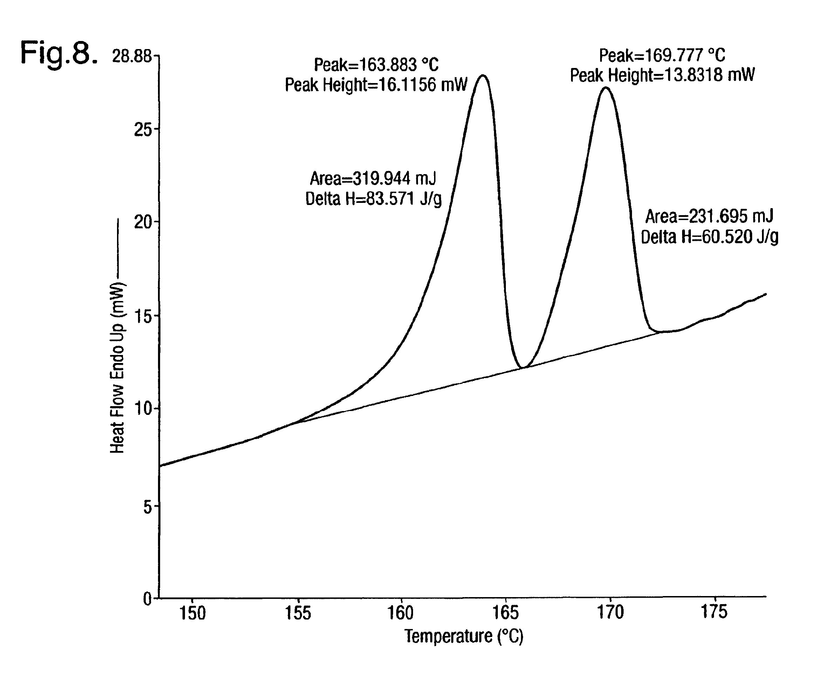

In the accompanying drawings Fig.5 illustrates the DSC analysis indicating a combination of Famotidine polymorph A and polymorph B in a specific ratio of 15:85.
In the accompanying drawings Fig.6 illustrates the DSC analysis indicating a combination of Famotidine polymorph A and polymorph B in a specific ratio of 05:95
In the accompanying drawings Fig.7 illustrates the DSC analysis indicating a combination of Famotidine polymorph A and polymorph B in a specific ratio of 27:73.
In the accompanying drawings Fig.8 illustrates the DSC analysis indicating a combination of Famotidine polymorph A and polymorph B in a specific ratio of 42:58.

The following examples are illustrated of the process of preparation of Famotidine pure polymorph A (of Fig.1), Famotidine pure polymorph B (of Fig.2) and a combination of Famotidine polymorph A and B (according to this invention) in a specific ratio of 22:78 of Fig.3, a combination of Famotidine polymorph A and B (according to this invention) in a specific ratio of 35:65 of Fig.4, a combination of Famotidine polymorph A and B (according to this invention) in a specific ratio of 15:85 of Fig.5 and a combination of Famotidine polymorph A and B (according to this invention) in a specific ratio of 5:95 of Fig.6, a combination of Famotidine polymorph A and B (according to this invention) in a specific ratio of 27:73 of Fig.7, a combination of Famotidine polymorph A and B (according to this invention) in a specific ratio of 42:58 of Fig.8.

### Example 1

In a 3000 ml, three necked round bottomed flask, equipped with a stirrer, thermometer and a reflux condenser, placed on a water bath, Methanol (2750 ml) is taken and Famotidine (crude) (50 g) is added into it. The mixture is heated to boiling point (75 C) and all the Famotidine is dissolved to give a solution. Activated carbon (20 g) is added into it and mixed well for 30 minutes. It is then filtered in a Buchner funnel to give a clear colorless solution. The solution is immediately transferred to another 3000 ml, three necked round bottomed flask, equipped with a stirrer, thermometer and cooled under agitation with cooling water. The temperature dropped to 25 C in about 2 hours time and it was cooled in ice + salt mixture to 0°C. The crystals of Famotidine thus obtained was filtered and dried in oven at 60° C.
The DSC analysis indicate it to be Famotidine Polymorph "A" Pure (Fig 1).

### Example 2

In a 3000 ml, three necked round bottomed flask, equipped with a stirrer, thermometer and a reflux condenser, placed on a water bath, Methanol (2750 ml) is taken and Famotidine (crude) (50 g) is added into it. The mixture is heated to boiling point (75 C) and all the Famotidine is dissolved to give a solution. Activated carbon (20 g) is added into it and mixed well for 30 minutes. It is then filtered in a Buchner funnel to give a clear colorless solution. The solution is immediately transferred to another 3000 ml, three necked round bottomed flask, equipped with a stirrer, thermometer and cooled under agitation with Ice + Salt mixture. The temperature dropped to 0 C in about 1 hour. The crystals of Famotidine thus obtained was filtered and dried in oven at 60° C.
The DSC analysis indicate it to be Famotidine Polymorph "B"Pure (Fig.2).

### Example 3

In a 3000 ml, three necked bottomed flask, equipped with a stirrer, thermometer and a reflux condenser, placed on a water bath, Methanol (2750 ml) is taken and Famotidine (crude) (50 g) is added into it. The mixture is heated to boiling point (75 C) and all the Famotidine is dissolved to give a solution. Activated carbon (20 g) is added into it and mixed well for 30 minutes. It is then filtered in a Buchner funnel to give a clear colorless solution. The solution is immediately transferred to another 3000 ml, three necked round bottomed flask, equipped with a stirrer, thermometer and cooled under agitation with Ice + Salt mixture. The temperature dropped to 20 C in about 1 hour and it was seeded with a mixture of Famotidine polymorphs A:B (20:80). The crystals of Famotidine thus obtained was filtered and dried in oven at 55 C.

The DSC analysis indicate it to be a mixture of Famotidine Famotidine Polymorph "A" and Famotidine Polymorph "B" (Fig. 2) (22:78) (Fig.3).

### Example 4

In a 3000 ml, three necked bottomed flask, equipped with a stirrer, thermometer and a reflux condenser, placed on a water bath, Methanol (2750 ml) is taken and Famotidine (crude) (50 g) is added into it. The mixture is heated to boiling point (75 C) and all the Famotidine is dissolved to give a solution. Activated carbon (20 g) is added into it and mixed well for 30 minutes. It is then filtered in a Buchner funnel to give a clear colorless solution. The solution is immediately transferred to another 3000 ml, three necked round bottomed flask, equipped with a stirrer, thermometer and cooled under agitation with ice + Salt mixture. The temperature dropped to 25 C in about 1 hour and it was seeded with a mixture of Famotidine polymorphs A:B (20:80). The crystals of Famotidine thus obtained was filtered and dried in oven at 55 C.

The DSC analysis indicate it to be a mixture of Famotidine Famotidine Polymorph "A" and Famotidine Polymorph "B" (Fig.2) (35:65) (Fig.4).

### Example 5

In a 3000 ml, three necked bottomed flask, equipped with a stirrer, thermometer and a reflux condenser, placed on a water bath, Methanol (2750 ml) is taken and Famotidine (crude) (50 g) is added into it. The mixture is heated to boiling point (75 C) and all the Famotidine is dissolved to give a solution. Activated carbon (20 g) is added into it and mixed well for 30 minutes. It is then filtered in a Buchner funnel to give a clear colorless solution. The solution is immediately transferred to another 3000 ml, three necked round bottomed flask, equipped with a stirrer, thermometer and cooled under agitation with ice + Salt mixture. The temperature dropped to 25 C in about 1 hour and it was seeded with a mixture of Famotidine polymorphs A:B (50:50). The crystals of Famotidine thus obtained was filtered and dried in oven at 55 C.

The DSC analysis indicate it to be a mixture of Famotidine Famotidine Polymorph "A" and Famotidine Polymorph "B" (Fig.2) (15:85) (Fig.5).

### Example 6

In a 3000 ml, three necked bottomed flask, equipped with a stirrer, thermometer and a reflux condenser, placed on a water bath, Methanol (2750 ml) is taken and Famotidine (crude) (50 g) is added into it. The mixture is heated to dissolve and all the Famotidine is dissolved to give a solution. Activated carbon (20 g) is added into it and mixed well for 30 minutes at 45°C . It is then filtered in a Buchner funnel to give a clear colorless solution. The solution is immediately transferred to another 3000 ml, three necked round bottomed flask, equipped with a stirrer, thermometer and concentrated under vacuum at 45-58°C to effect Evaporative crystallization. After distilling about 33% of the solvent, a crystalline slurry was obtained, which was filtered at 45°C .The crystals of combination of Famotidine polymorph" A" and Famotidine Polymorph "B". Famotidine, thus obtained was dried in oven at 60° C. (DSC analysis : Fig.6).

The DSC analysis indicate it to be a mixture of F Famotidine Polymorph "A" and Famotidine Polymorph "B" (05:95) (Fig.6).

### Example 7

In a 3000 ml, three necked bottomed flask, equipped with a stirrer, thermometer and a reflux condenser, placed on a water bath, Methanol (2750 ml) is taken and Famotidine (crude) (50 g) is added into it. The mixture is heated to dissolve and all the Famotidine is dissolved to give a solution. Activated carbon (20 g) is added into it and mixed well for 30 minutes at 45°C. It is then filtered in a Buchner funnel to give a clear colorless solution. The solution is immediately transferred to another 3000 ml, three necked round bottomed flask, equipped with a stirrer, thermometer and concentrated under vacuum at 45-58°C to effect Evaporative crystallization. After distilling about 90% of the solvent, a crystalline slurry was obtained, which was filtered at 45°C .The crystals of combination of Famotidine polymorphs"A" and "B" Famotidine thus obtained was dried in oven at 60° C.

The DSC analysis indicate it to be a mixture of Famotidine Famotidine Polymorph "A" and Famotidine Polymorph "B" (27:73) (Fig.7).

### Example 8

In a 3000 ml, three necked bottomed flask, equipped with a stirrer, thermometer and a reflux condenser, placed on a water bath, Methanol (500 ml) is taken and Famotidine (crude) (50 g) is added into it. Hydrochloric acid was added and this mixture was heated to 40°C to precipitate the Famotidine hydrochloride. The Famotidine Hydrochloride thus obtained was filtered in a Buchner funnel. The Famotidine Hydrochloride and Methanol 2750 ml were heated to dissolve and all the Famotidine hydrochloride is dissolved to give a solution. Activated carbon (20 g) is added into it and mixed well for 30 minutes at 45°C.. It is then filtered in a Buchner funnel to give a clear colorless solution. The solution is immediately transferred to another 3000 ml, three necked round bottomed flask, equipped with a stirrer, thermometer and made basic using Triethylamine (60 ml) concentrated under vacuum to effect evaporative crystallization, at 45-58°C. After distilling about 70 % of the solvent methanol, a crystalline slurry was obtained, which was filtered in a Buchner funnel. The crystals of Famotidine thus obtained was filtered and dried in oven at 60° C.

The DSC analysis indicate it to be a mixture of Famotidine Polymorph "A" and Famotidine Polymorph "B" (Fig.5) (42:58) (Fig.8).

The above description with reference to examples has been given just to understand the invention rather than to limit its scope.

## Claims

1. A process for the preparation of a combination of Famotidine Polymorphs A and B comprising the following steps:
a) dissolving Famotidine crude in a solvent under heating and stirring to form a solution;
b) filtering the solution of step (a);
c) cooling the solution of step (b) with an ice and salt mixture under agitation;
d) seeding the cooled solution of step (c) with a mixture of Famotidine polymorph A and Famotidine Polymorph B for crystallization;
e) filtering out the crystals of Famotidine Polymorphs A and B;
f) drying the crystals of Famotidine Polymorphs A and B.

2. A process as claimed in claim 1, wherein Famotidine (crude) to solvent ratio is 1:50 to 1:70.

3. A process for the preparation of a combination of Famotidine Polymorphs A and B comprising the following steps:
(a) dissolving Famotidine crude in a solvent under heating and stirring to form a solution;
(b) filtering the solution of step (a);
(c) concentrating the solution by heating under vacuum to get a crystalline slurry;
(d) filtering out the crystals of Famotidine Polymorphs A and B;
(e) drying the crystals of Famotidine Polymorphs A and B.

4. A process as claimed in claim 3, wherein Famotidine (crude) to solvent ratio is 1:50 to 1:70.

5. A process as claimed in claim 3 wherein activated carbon is added to the solution at step (a) before the solution is filtered in step (b).

6. A process as claimed in claim 3, wherein at step (c) the solution is concentrated at a temperature around 45°C to 58°C.

7. A process for the preparation of a combination of Famotidine Polymorphs A and B comprising the following steps:
(a) dissolving Famotidine crude in a solvent under heating and stirring and reacting with concentrated Hydrochloric acid to give Famotidine Hydrochloride;
(b) dissolving the Famotidine Hydrochloride in solvent;
(c) filtering the solution of step (b);
(d) making the solution of step (c) basic with triethylamine;
(e) concentrating the solution of step (d) by heating under vacuum to give a crystalline slurry;
(f) filtering out the crystals of Famotidine Polymorphs A and B;
(g) drying the said crystals of Famotidine Polymorphs A and B.

8. A process as claimed in claim 7, wherein the Famotidine Hydrochloride to solvent ratio is 1:50 to 1:70.

9. A process as claimed in claim 7 wherein activated carbon is added to the solution of step (b) before the solution is filtered in step (c).

10. A process as claimed in claim 7, wherein at step (e) the solution is concentrated at a temperature of around 45°C to 58°C.
